# EUROPEAN PATENT SPECIFICATION

(11) **EP 3 393 407 B1**
(45) Date of publication and mention of the grant of the patent: **26.11.2025**
(21) Application number: 16880029.0
(22) Date of filing: 21.12.2016
(51) Int. Cl.: A61F 2/86, A61L 31/14, A61L 31/02

(54) **PROCESS TO PRODUCE HIGH-STRENGTH AND CORROSION RESISTANT ALLOY FOR PATIENT-SPECIFIC BIORESORBABLE BONE FIXATION IMPLANTS AND HARDWARE**
VERFAHREN ZUR HERSTELLUNG VON HOCHFESTEN UND KORROSIONSBESTÄNDIGEN LEGIERUNGEN FÜR PATIENTENSPEZIFISCHE BIORESORBIERBARE KNOCHENFIXATIONSIMPLANTATE UND HARDWARE
PROCÉDÉ DE PRODUCTION D'ALLIAGE À HAUTE RÉSISTANCE ET RÉSISTANT À LA CORROSION POUR IMPLANTS ET MATÉRIEL DE FIXATION OSSEUSE BIORÉSORBABLE SPÉCIFIQUES À UN PATIENT

(30) Priority: 21.12.2015 US 201562270227 P
(43) Date of publication of application: 31.10.2018
(73) Proprietor: The University of Toledo, Toledo, OH 43606 (US)
(72) Inventor: IBRAHIM, Hamdy, Toledo OH 43606 (US); ELAHINIA, Mohammad, Sylvania OH 43560 (US)
(74) Representative: Casalonga
(86) International application number: PCT/US2016/068038
(87) International publication number: WO 2017/112779

(56) References cited:
- WO-A1-2014/145672
- CN-A- 101 392 344
- CN-A- 101 629 260
- US-A1- 2006 045 787
- US-A1- 2011 189 377
- WEI KAIWEN ET AL: "Effect of energy input on formability, microstructure and mechanical properties of selective laser melted AZ91D magnesium alloy", MATERIALS SCIENCE, vol. 611, 9 June 2014 (2014-06-09), pages 212 - 222, XP029034607, ISSN: 0921-5093, DOI: 10.1016/J.MSEA.2014.05.092
- KARUNAKARAN RAKESHKUMAR ET AL: "Additive manufacturing of magnesium alloys", BIOACTIVE MATERIALS, vol. 5, no. 1, 1 March 2020 (2020-03-01), pages 44 - 54, XP055860570, ISSN: 2452-199X, DOI: 10.1016/j.bioactmat.2019.12.004

## Description

### CROSS REFERENCE TO RELATED APPLICATION

The present patent application is based upon and claims the benefit of provisional patent application No. 62/270,227 filed December 21, 2016.

### BACKGROUND OF THE INVENTION

This invention relates to a biocompatible Mg-Zn-Ca-based alloy and a post-shaping heat treatment process for the production of patient-specific bioresorbable bone fixation hardware.

Mg-Zn-Ca-based alloys are the most promising alloy system for bone implant applications mainly due to their superior biocompatibility. Wei Kalwen et al. "Effect of energy imput on the formability, microstructure and mechanical properties of selective laser melted AZ91D magnesium alloy" in Material Science; vol. 611, pages 212-222 discloses that selective laser melting (SLM) technology has been used to manufacture the AZ91D magnesium alloys. The alloys find use in the medical field. AZ91D parts with complex structures and high performance are increasing fast in number to challenge conventional manufacturing methods. SLM technique can be used to solve the manufacturing problems of such complex structures. Spherical gas atomized AZ91D powders were used as the starting material in SLM experiments. For patient-specific (3D-printed) fixation hardware made of an Mg-Zn-Ca-based alloy, heat treatment can be used to improve the mechanical properties of such alloys by means of intermetallic precipitations.

Some efforts have been made to improve the mechanical and/or corrosion properties of metallic alloys using different heat or mechanical treatment processes. As an example, Kaese et al., as described in US Pat. No. 6,854,172, B2, used casting process, heat treatment, particularly homogenization, a subsequent thermomechanical treatment, particularly extrusion and finally cutting process to produce a pin-shaped thin-walled tubular implant for cardiovascular supports made of Mg or Zn alloys.

For Mg-Zn-Ca based alloys, different heat treatment methods have been done to enhance their mechanical or corrosion properties such as annealing, solution treatment, quenching and age hardening. Most of them were mainly performed to enhance the mechanical properties of such alloys mainly for structural applications without investigating the effect of heat treatment on the biocompatibility and biocorrosion properties of these alloys. For example, the method followed in the work of Oh-Ishi et al. entitled "Age-Hardening Response of Mg-0.3 at %Ca Alloys with Different Zn Contents' was focused only on enhancing the hardness as a measure of mechanical properties of the Mg-0.5wt%Ca alloys with 0 to 4.2 wt.%Zn.

Only two efforts paid attention to the effect of heat treatment on the biocorrosion behavior of Mg-Zn-Ca-based alloys as a potential bone fixation application. The first effort is the work of Lu et al. entitled "Effects of Secondary Phase and Grain Size on the Corrosion of Biodegradable Mg-Zn-Ca Alloys". In this work, only solution treatment and quenching were performed on Mg-3wt.%Zn-0.3wt.%Ca alloy without performing any age hardening process. They found an enhancement in the alloy's biocorrosion resistance after solution treatment and quenching. However, the resulting alloy's mechanical properties after heat treatment were not investigated.

The second effort is the work of Ji et al. entitled "Influence of Heat Treatment on Biocorrosion and Hemocompatibility of Biodegradable Mg-35Zn-3Ca Alloy". An enhancement in the biocorrosion resistance and biocompatibility of the heat-treated alloy was found. However, the effect of the heat treatment process on the mechanical properties for the studied alloy (Mg-35wt. %Zn-3wt. %Ca) was again ignored, similar to the previously mentioned efforts. In addition, the studied alloy in the Ji et al. work has significantly high content of alloying elements especially Zn (35 wt.%). It has been reported in several studies that high loading of Zn causes an excess formation of Ca₂Mg₆Znₐ secondary phase which suppresses the formation of the finely dispersed monolayer Guinier-Preston (G.P.) zones leading to lower age hardening effect, hence less mechanical properties enhancement.

In general, using heat treatment for developing Mg-Zn-Ca-based bone fixations should consider the following aspects: (i) proper choice of the alloy chemical composition, (ii) proper choice of the heat treatment processes and parameters, (iii) assessment of mechanical properties and (iv) assessment of biocorrosion properties after heat treatment. None of the previous heat treatment methods covered all these needed aspects.

### SUMMARY OF THE INVENTION

Accordingly, an object of subject invention is to carefully cover these aspects to provide a process for producing a Mg-Zn-Ca-based alloy for bone fixation hardware.

Another object is the development of a novel Mg-Zn-Ca-based alloy and a heat treatment method that, in addition to its role in improving the Mg-Zn-Ca-based alloy's mechanical properties, also significantly enhances its biocorrosion properties.

This enhancement in the biocorrosion properties is represented in a significantly slower degradation rate and a more uniform (predicted) degradation of the heat-treated Mg-Zn-Ca alloy compared to the as-cast one in a simulated body fluid medium (in-vitro tests). The slower in-vitro degradation rate and higher strength of the heat-treated alloy indicates that this heat treatment method is a promising post-shaping process for the prepared Mg-Zn-Ca-based alloy in order to produce biocompatible, strong and less biocorrosive devices for bone fixation and applications.

Still another subject improvement and preferred embodiments of the invention are described in detail hereinafter.

Other objects and advantages of the present invention will become apparent to those skilled in the art upon a review of the following detailed description of the preferred embodiments and the accompanying drawings.

### BRIEF DESCRIPTIONS OF THE DRAWINGS

Figs. 1a through 1f are SEM micrographs of the as-cast compared against the heat-treated alloy.
Figs. 2a and 2b are optical micrographs of the as-cast Mg-Zn-Ca alloy without Mn compared against the as-cast Mg-Zn-Ca alloy with Mn.
Fig. 3a and 3b are SEM micrographs at different magnifications of the ceramic coating created on the heat-treated alloy using MAO.
Fig. 4 is a graph showing mechanical properties.
Fig. 5 is a graph showing polarization curves of as cast versus the heat-treated alloy.
Fig. 6 are photographs of the corroded alloy after *in vitro* immersion.
Fig. 7 is a graph of *in vitro* corrosion properties of the alloy of as cast versus the heat-treated alloy.
Fig. 8 is a graph showing mechanical properties.
Fig. 9 is a graph showing polarization curves of the heat-treated alloy.
Fig. 10 is a graph showing mechanical properties of as cast versus the heat-treated alloy with the addition of Mn.
Fig. 11 is a graph showing polarization curves of the heat-treated alloys with the addition of Mn.
Fig. 12 is a graph showing polarization curves of the heat-treated alloy and the MAO coated alloy.
Fig. 13 is a graph of *in vitro* corrosion properties of the different alloys.
Fig. 14 is a graph of an XRD analysis of the alloy before and after immersion.

### DETAILED DESCRIPTION OF THE INVENTION

This invention proposes a process in which a Mg-Zn-Ca-based alloy is cast and then heat-treated, particularly solution-treated, quenched and age-hardened. The invention relates to a patient-specific bioresorbable magnesium (Mg) based bone fixation hardware made of a cast alloy consisting of: 5 to 2.0 percent by weight of zinc (Zn); 5 to 1.0 percent by weight of calcium (Ca); 5 to 1.0 percent by weight of manganese (Mn); remainder being magnesium (Mg), wherein the Zn/Ca atomic ratio of the prepared alloy is within the range of 1.2 to 1.47; and wherein the cast alloy is heat-treated and atomized into powder and patient-specific fixation hardware is produced by 3D-printing. In the preferred embodiment, the Mg-Zn-Ca-based alloy contains Mg-1.2wt%Zn-0.5wt.%Ca. The Mg-Zn-Ca-based alloy was prepared by using commercial Mg, pure Zn, and a 15% Ca-Mg master alloy.

The enhancement in the mechanical properties of Mg-Zn-Ca-based alloys after heat treatment is due to the refinement and uniform distribution of the (α-Mg+Ca₂Mg₆Zn₃) eutectic phase into fine dispersions precipitates. Therefore, the chemical composition of the cast Mg-Zn-Ca alloy must be carefully chosen to obtain the optimum age hardening effect after the heat treatment process. For instance, a Ca content of 0.5wt% (less than 1 wt.%, the solubility limit of Ca in Mg) will avoid excess formation of Mg₂Ca phase that hinders the age hardening process. Further, any increase in the Ca content above the solubility limit does not result in more grain refinement. A Zn content of 1.2 wt.% will avoid excess formation of Ca₂Mg₆Zn₃ phase since a high concentration of Ca₂Mg₆Zn₃ phase suppresses the formation of the finely dispersed monolayer Guinier-Preston (G.P.) zones on basal planes leading to a less age hardening effect. Further, a relatively low (below 5 wt.%) Zn content is preferred to avoid formation of the MgZn phase that has no role in the age hardening effect.

This means that the designed Zn/Ca atomic ratios of the prepared alloy (1.47) is within the range of (1.2 to 2.0) for the optimum corrosion properties and age hardening effect The alloys chemical composition and the level of impurities were evaluated via two different techniques: X-ray florescence (XRF) and energy dispersive spectroscopy (EDS) and they were found to be similar to the designed values.

Heat treatment processes and parameters were chosen carefully to obtain the optimum results. For instance, the as-cast alloy was solution-treated above solidus line at 510 °C for 3 h in an inert gas environment using a tube furnace to assure the melting of Mg₂Ca and Ca₂Mg₆Zn₃ eutectic phases in the primary α-Mg matrix. The solution-treated alloy was then quenched in cold water to trap fine dispersions of the Ca₂Mg₆Zn₃ intermetallic phase in the primary α-Mg matrix. Afterwards, the quenched alloy was artificially age-hardened in an oil bath at 200 °C to uniformly distribute the thermally stable (α-Mg+Ca₂Mg₆Zn₃) and/or (α-Mg+Mg₂Ca+Ca₂Mg₆Zn₃) intermetallic phase fine dispersions. The alloy was aged for different durations (1, 2, 3. 5 and 10 hours) to determine which duration produced the optimum age hardening effect on the mechanical and corrosion behavior for bone fixation applications. Similarly, the alloy was aged at different age hardening temperatures (100, 150, 200 and 250 °C) to determine the aging temperature that results in the highest mechanical and corrosion resistance.

The effect of adding 0.5 wt. % Mn on the mechanical and corrosion properties of the ternary Mg-1.2wt%Zn-0.5wt. %Ca alloy was investigated, as well. Finally, the heat-treated Mg-Zn-Ca-based alloy at the optimum conditions was coated with a biocompatible ceramic coating using a micro arc oxidation (MAO) process for slower and more tailored biocorrosion rates.

The as-cast and heat-treated ingots were cut into the needed sample sizes and carefully prepared for their mechanical and corrosion properties characterization.

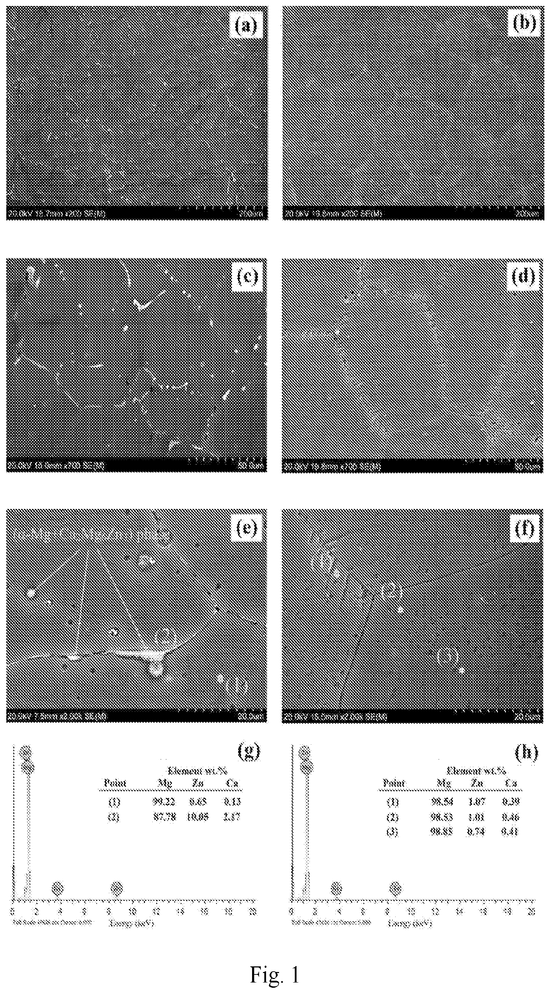

Figures 1.a., 1.c. and 1.e. show the scanning electron microscope (SEM) micrographs of the as-cast alloy. The formation of the (α-Mg+Ca₂Mg₆Zn₃) secondary phase can be observed as lamellar eutectoids within grain boundaries of the primary α-Mg matrix phase and spherical precipitates within the α-Mg matrix interdendritic interstices. This observation was supported by results from the EDS analysis, as seen in Figure 1.g. The solid solution (point 1) was found to be Mg-rich solution with low content of Zn and Ca which indicates that it is the primary α-Mg phase. The secondary phase (point 2) has a significant higher content of Zn and Ca which indicates that it is the eutectic (α-Mg+Ca₂Mg₆Zn₃) phase.

Figures 1.b., 1.d. and 1.f. show the SEM micrographs of the heat-treated alloy. It can be seen that the grains have a dendritic structure with relatively larger grain size compared to the as-cast alloy. Also, the (α-Mg+Ca₂Mg₆Zn₃) secondary phase was refined and converted into uniformly distributed and finely dispersed precipitates within the microstructure after heat-treatment. The results obtained from the EDS analysis for the heat-treated microstructure, Figure 1.h., show similar Mg, Zn and Ca contents at different investigated points within the microstructure which confirms the uniform distribution of Ca₂Mg₆Zn₃ into finely dispersed precipitates. It is well-known that the refinement of secondary phases and the formation of the finely dispersed monolayer Guinier-Preston (G.P.) zones on basal planes are responsible for the age hardening effect after heat treatment

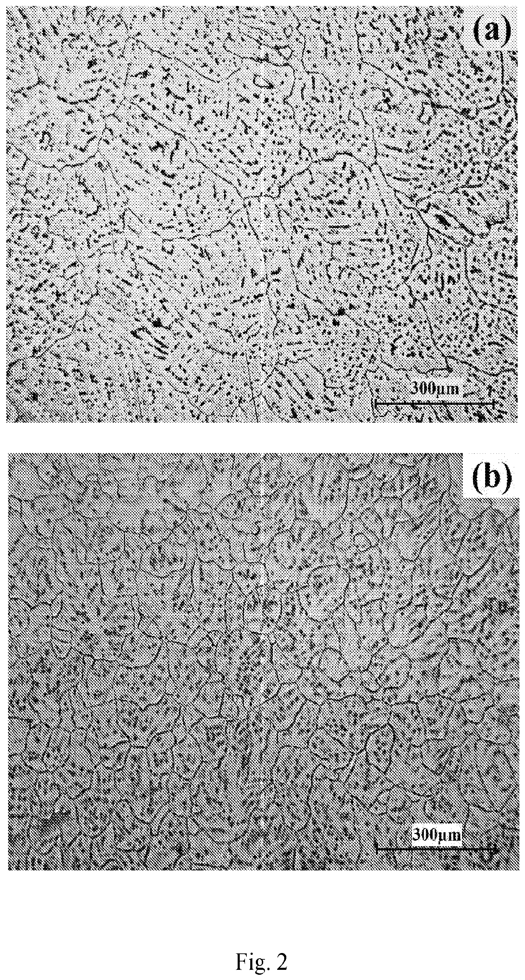

Figures 2.a. and 2.b. show the optical micrographs of the as-cast Mg-1.2wt%Zn-0.5wt.%Ca alloy in comparison to the Mg-1.2wt%Zn-0.5wt.%Ca-0.5wt.%Mn alloy, respectively. The grain size of the alloy after the addition of Mn was much lower than that for the Mn-free alloy. The average grain size of the Mg-1.2wt%Zn-0.5wt.%Ca alloy and the Mg-1.2wt%Zn-0.5wt.%Ca-0.5wt.%Mn alloy was around 216 µm and 67 µm, respectively. Such grain refinement effect is expected to result in an improved mechanical and corrosion properties for the Mg alloy before and after heat treatment.

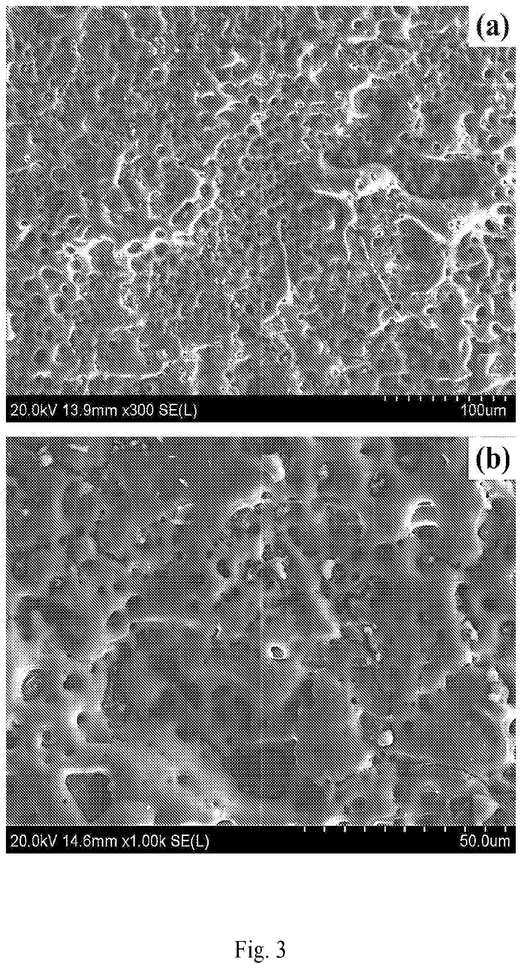

Figure 3 shows the SEM micrographs of a ceramic coating prepared on the heat-treated Mg-Zn-Ca-based alloy using a MAO process.

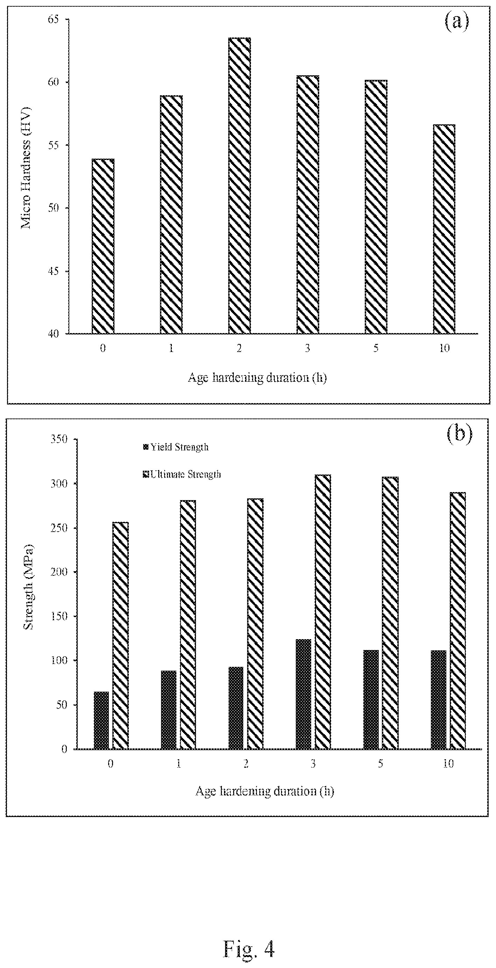

Figure 4 shows the mechanical properties (micro-hardness and compression) of the as-cast and heat-treated alloy aged at 200 °C. The heat treatment process was found to significantly increase the alloy mechanical properties. Age hardening duration of 2-5 hours showed the optimum mechanical properties. For instance, the tensile and compressive yield strengths of the alloy age-hardened for 3 hours were 1.4 and 1.9 times those for the as-cast alloy, respectively.

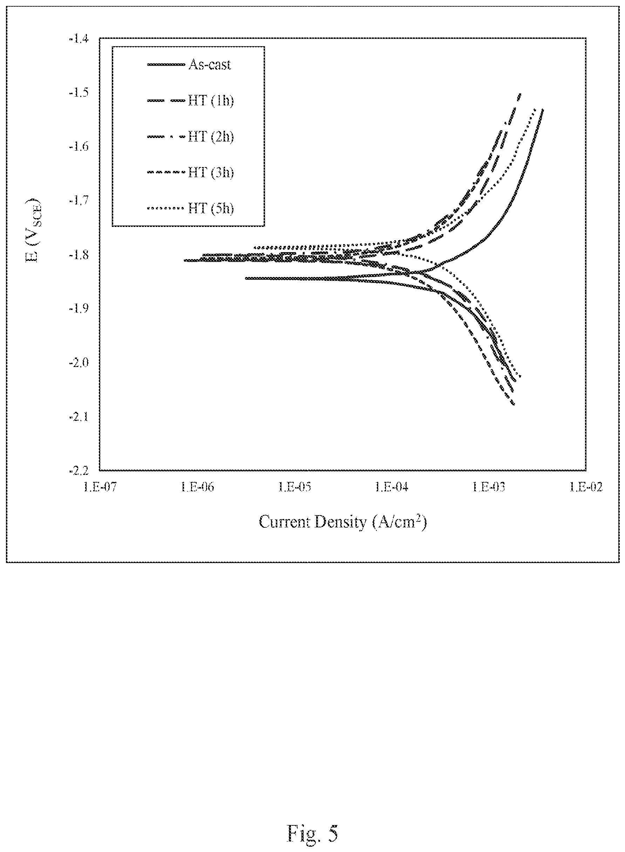

The polarization curves for the as-cast and heat-treated alloy tested in m-SBF solution at pH 7.4 and 37 °C are shown in Figure 5. The heat-treated alloy has less negative potential and lower corrosion density than the heat-treated alloy. This indicates that the heat-treated alloy has better electrochemical corrosion characteristics that results in a lower corrosion rate (P) for the heat-treated alloy than the as-cast alloy, as seen in table 1. The best corrosion resistance was obtained for alloy samples aged for 2-5 hours.

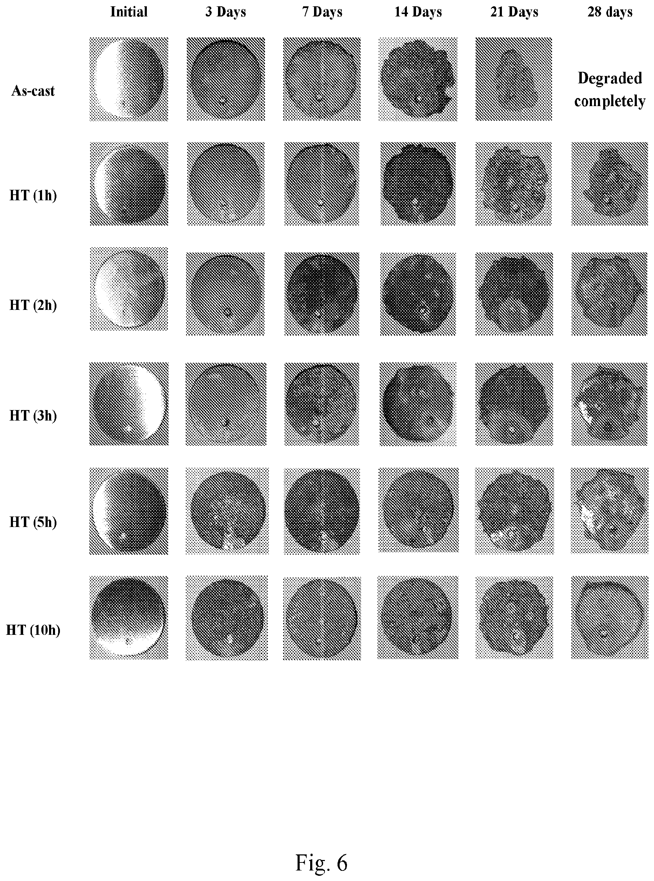

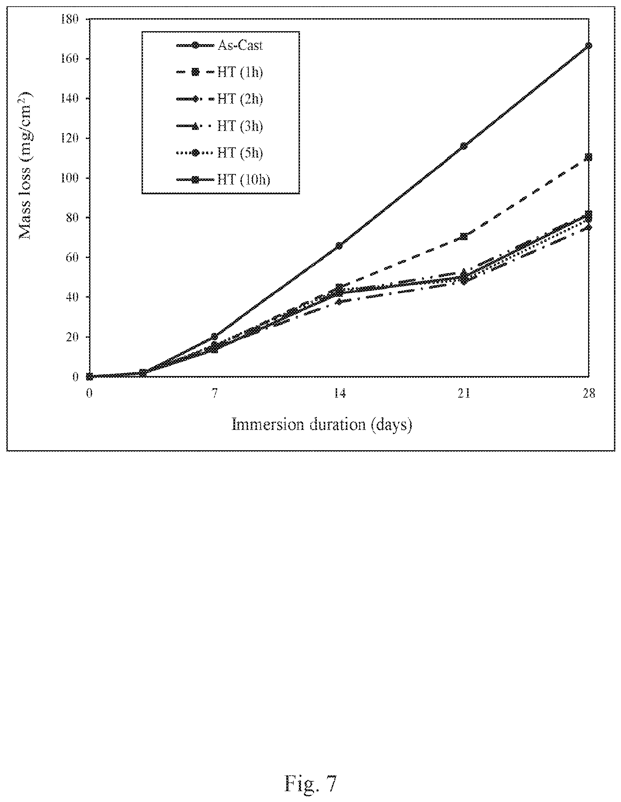

Figure 6 shows optical images of degraded as-cast and heat-treated alloy after *in vitro* immersion in m-SBF at 37 °C and 7.3-7.8 pH for 3, 7, 14, 21 and 28 days. Both as-cast and heat-treated alloy samples showed a small amount of degradation (less than 2 mg/cm²) after immersion for 3 days. However, with increasing the immersion duration (more than 7 days), the degradation of the as-cast alloy increased remarkably compared to the heat-treated alloy samples. On contrary to the heat-treated alloy samples, the as-cast samples showed uneven surface with large cracked regions (pitting corrosion) that were subjected to intense corrosion attack. This is suggested to be due to the presence of larger regions of the (α-Mg+Ca₂Mg₆Zn₃) secondary phase that promotes the galvanic corrosion for the as-cast alloy than the heat-treated alloy. By day 28 of the *in vivo* immersion test, the as-cast alloy samples were degraded completely, while more than 50 % of the heat-treated alloy samples (2-10 age hardening durations) remained, see table 2. The age-hardened alloy samples for more than 2 hours have the superior corrosion resistance, see Figure 6, 7. For example, the corrosion rate of the heat-treated alloy aged for 2 to 5 hours was almost half that for the as-cast alloy.

The enhancement in the corrosion resistance of the Mg-Zn-Ca alloy after heat treatment can be attributed to the refinement and fine dispersion of the Ca₂Mg₆Zn₃ phase in the heat-treated alloy. Large precipitates of Ca₂Mg₆Zn₃ phase in the microstructure, as in the case of as-cast alloy, work as cathodic site for the α-Mg matrix phase, hence faster corrosion rate for the as-cast alloy is expected.

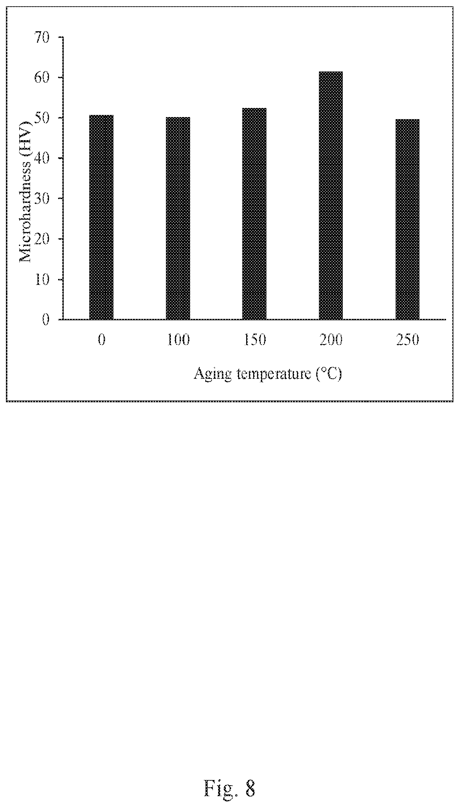

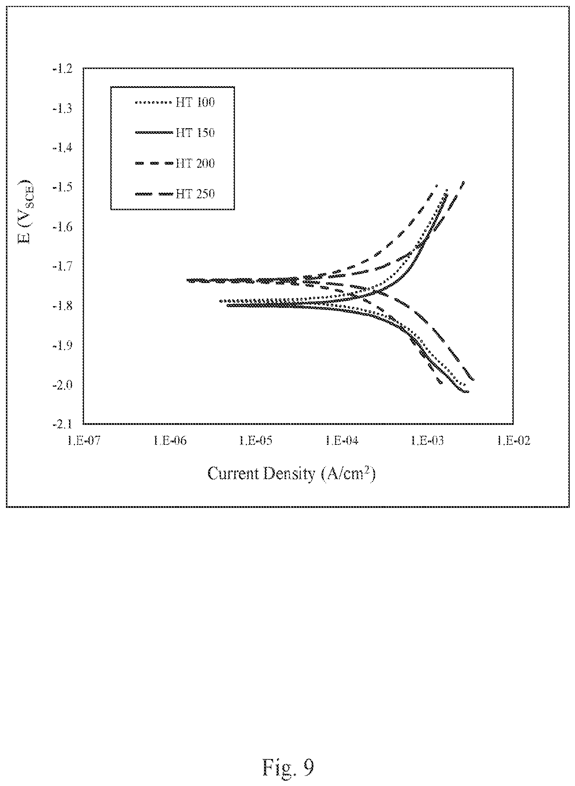

Figure 8 shows the effect of aging temperatures on the age hardening response (microhardness) of the heat-treated Mg-1.2wt%Zn-0.5wt.%Ca alloy aged for 3 hours and different aging temperatures. It can be seen that aging the Mg-Zn-Ca-based alloy at 200 °C results in the best age hardening response. Similarly, the effect of aging temperatures on the electrochemical corrosion properties at pH 7.4 and 37 °C of the heat-treated Mg-1.2wt%Zn-0.5wt.%Ca alloy is shown in Figure 9, while table 3 summarizes the electrochemical corrosion characteristics. It is obvious that aging the Mg-1.2wt%Zn-0.5wt.%Ca alloy at 200 °C results in the best corrosion behavior represented in the lowest corrosion rate.

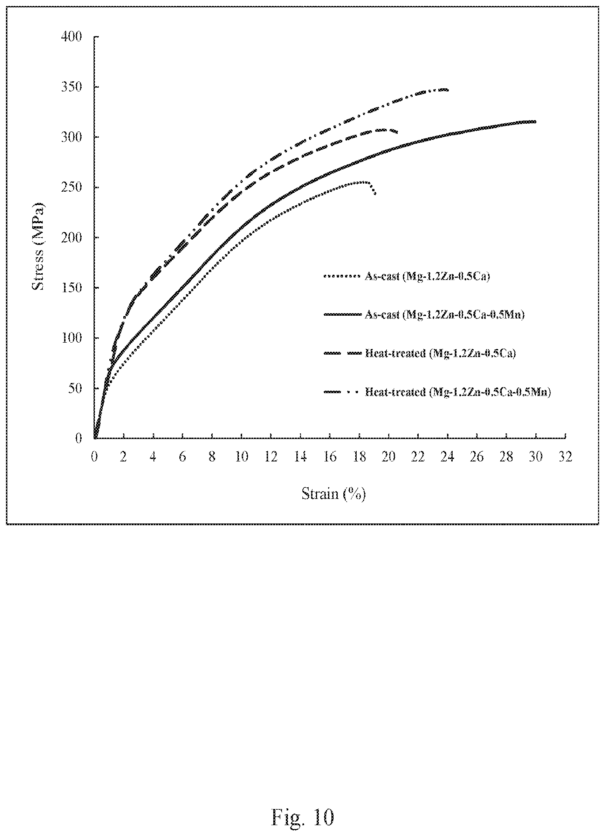

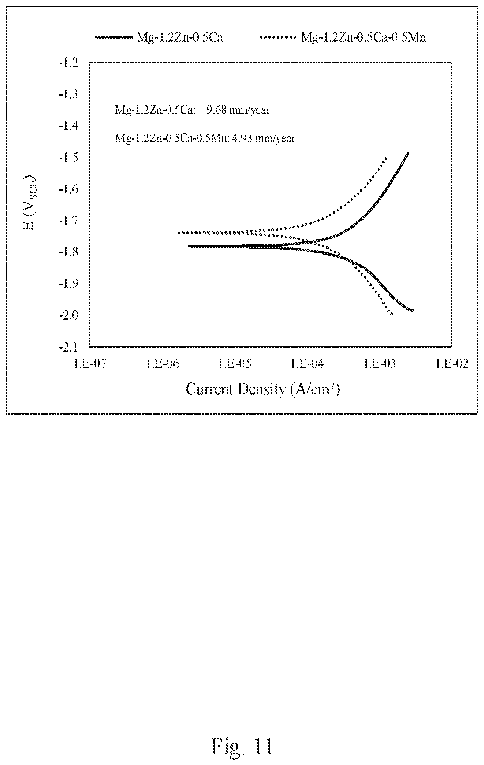

Figure 10 shows the effect of adding Mn on the compression stress-strain diagram of the as-cast and heat-treated Mg-Zn-Ca-based alloys. It can be seen that the age hardening response of the Mg-1.2wt.%Zn-0.5 wt.%Ca-0.5wt.%Mn alloy was higher than that for the Mg-1.2wt.%Zn-0.5 wt.%Ca alloy. The in vitro electrochemical corrosion test results at pH 7.4 and 37 °C showed significantly lower corrosion rates for the heat-treated Mg-Zn-Ca-based alloy after the addition of 0.5 wt.% Mn, as seen in Figure 11.

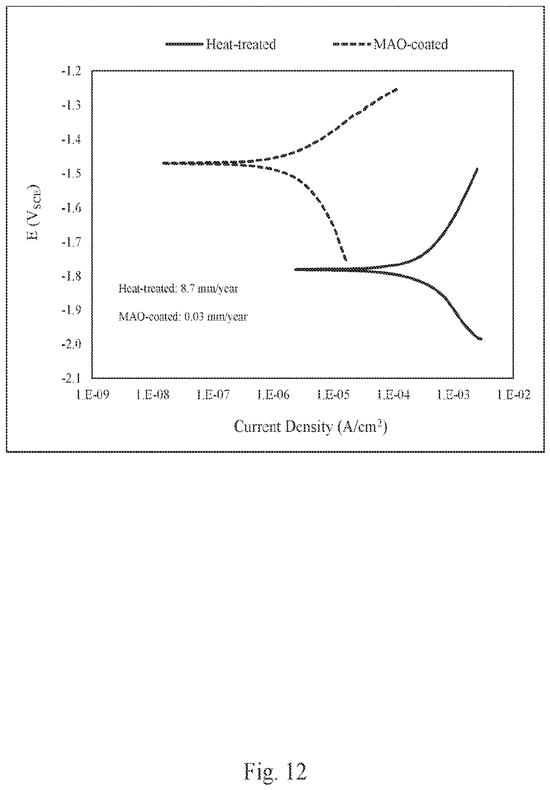

The electrochemical corrosion curves of the MAO-coated alloy at pH 7.4 and 37 °C after performing the heat-treatment process at the optimized parameters are shown in Figure 12. It can be seen that the corrosion rate of the heat-treated Mg-Zn-Ca-based alloy was significantly reduced from 8.7 mm/year to be 0.03 mm/year after the coating process.

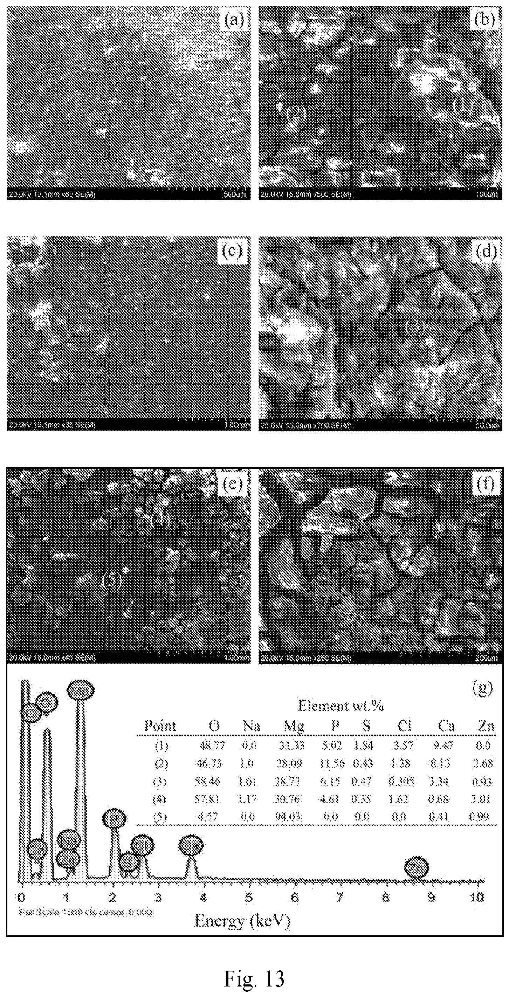

The morphologies and the elemental compositions of the as-cast and heat-treated alloy sample degraded surfaces after 3 and 28 days of immersion at 37 °C and 7.3-7.8 pH were studied using SEM investigation and the EDS analysis as shown in Figure 13. All samples showed uneven surface morphology by the 3 day of immersion with corrosion product agglomerations on the surface. The EDS elemental analysis of the agglomerated substance and the ground of the as-cast alloy surface showed large amounts of oxygen, calcium and phosphorus. The heat-treated alloy sample surface showed similar trend for the agglomerated substance. However, no traces of phosphorus on the ground of the corroded surface.

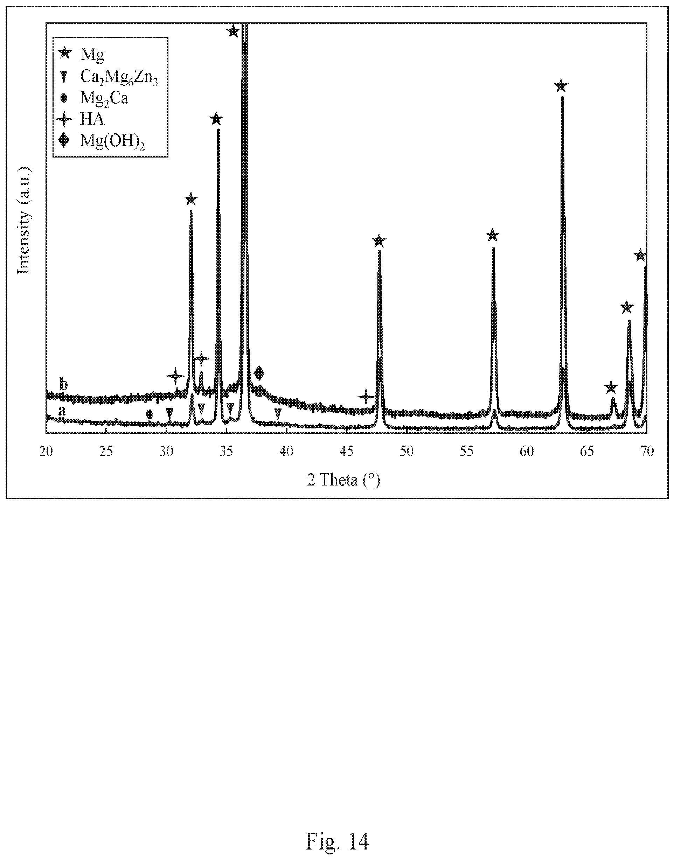

Figure 14 shows the XRD analysis for the heat-treated alloy samples (a) before immersion and (b) after immersion in m-SBF solution at 37 °C and 7.3-7.8 pH for 3 days. The XRD pattern of alloy before immersion showed the presence of small reflections of Mg₂Ca phase, which do not support its presence, and relatively higher intensity of Ca₂Mg₆Zn₃ phase in addition to Mg reflections. These results are in consistent with the microstructural investigation results. The XRD patterns for the alloy after immersion showed the presence of hydroxyapatite (HA) and magnesium hydroxide (Mg(OH)₂) as the main corrosion compounds formed on the surface of the corroded samples. Such biocompatible corrosion products, in addition to its role in bone growth stimulation, it may also act as a corrosion barrier that decelerate corrosion.

It is, therefore, to be understood that the invention may be practiced within the scope of the subjoined claims.

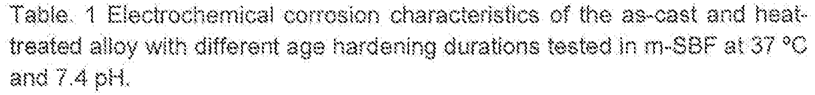

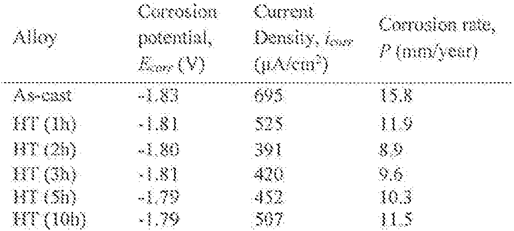

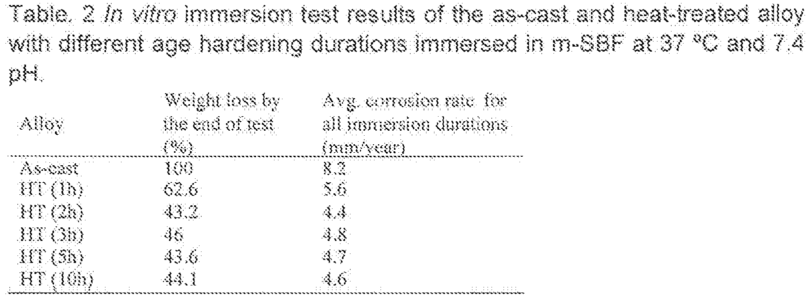

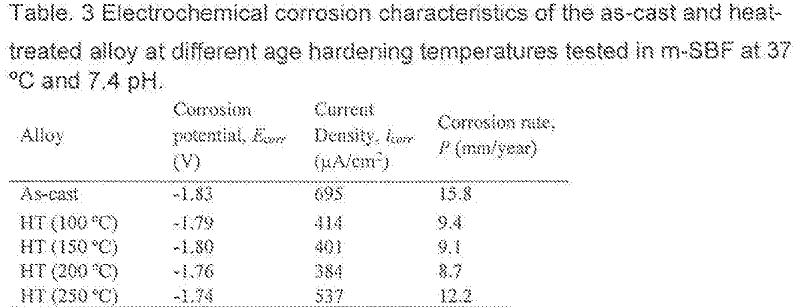

The above detailed description of the present invention is given for explanatory purposes. Accordingly, the whole of the foregoing description is to be construed in an illustrative and not a limitative sense, the scope of the invention being defined solely by the appended claims.

## Claims

1. A patient-specific bioresorbable magnesium (Mg) based bone fixation hardware made of a cast alloy consisting of:
0.75 to 2.0 percent by weight of zinc (Zn);
0.25 to 1.0 percent by weight of calcium (Ca);
0.25 to 1.0 percent by weight of manganese (Mn);
remainder being magnesium (Mg),
wherein the Zn/Ca atomic ratio of the prepared alloy is within the range of 1.2 to 1.47; and
wherein the cast alloy is heat-treated and atomized into powder and patient-specific fixation hardware is produced by 3D-printing.

2. The magnesium (Mg) based bone fixation hardware according to claim 1 wherein the cast alloy contains:
1.2 percent by weight of zinc (Zn);
0.5 percent by weight of calcium (Ca);
0.5 percent by weight of manganese (Mn); and
remainder being magnesium (Mg).

3. The magnesium (Mg) based bone fixation hardware according to claim 1 wherein the corrosion rate of the heat-treated alloy is at-least 40% lower than that of the untreated as-cast alloy.

4. The magnesium (Mg) based bone fixation hardware according to claim 1 wherein the corrosion rate of the heat-treated alloy is at-least 50% lower than that of the untreated as-cast alloy.

5. A process for producing patient-specific bioresorbable magnesium (Mg) based bone fixation hardware comprising the steps of:
producing patient-specific fixation hardware made of an alloy of:
0.75 to 2.0 percent by weight of zinc (Zn);
0.25 to 1.0 percent by weight of calcium (Ca);
0.25 to 0.75 percent by weight of manganese (Mn);
remainder being magnesium (Mg); and
wherein the Zn/Ca atomic ratio of the prepared alloy is within the range of 1.2 to 1.47;
heat treating the alloy,
wherein the alloy is produced by casting, and
wherein the cast alloy is atomized into powder and patient-specific fixation hardware is produced by 3D-printing.

6. The process according to claim 5 wherein the heat-treating step is solution treating, quenching or age hardening.

7. The process according to claim 5, wherein the heat-treated alloy possesses improved mechanical and biocorrosion properties.

8. The process according to claim 6 wherein the age hardening is carried out for 2 to 5 hours at 200 °C and the heat-treated alloy possesses half the corrosion rate of the as-cast alloy.

9. The process according to claim 6 wherein the age hardening is carried out for 3 to 5 hours at 200 °C and the heat-treated alloy possesses half the corrosion rate of the as-cast alloy.

10. The process according to claim 7 wherein the heat-treated alloy with optimum mechanical and biocorrosion properties is further coated with a ceramic coating using micro arc oxidation process.

11. The process according to claim 9 wherein the alloy has biocompatible corrosion byproducts of hydroxyapatite (HA) and magnesium hydroxide (Mg(OH)₂).

12. The magnesium (Mg) based bone fixation hardware obtainable with the process of claim 8 or 9.

## Patentansprüche

1. Patientenspezifische, bioresorbierbare Knochenfixierungsvorrichtungen auf Basis von Magnesium (Mg), hergestellt aus einer Gusslegierung, bestehend aus:
0,75 bis 2,0 Gewichtsprozent Zink (Zn);
0,25 bis 1,0 Gewichtsprozent Kalzium (Ca);
0,25 bis 1,0 Gewichtsprozent Mangan (Mn);
wobei der Rest Magnesium (Mg) ist,
wobei das Zn/Ca-Atomverhältnis der hergestellten Legierung im Bereich von 1,2 bis 1,47 liegt; und
wobei die Gusslegierung wärmebehandelt und zu Pulver zermahlen wird und patientenspezifische Fixierungsvorrichtungen durch 3D-Druck hergestellt werden.

2. Knochenfixierungsvorrichtungen auf Basis von Magnesium (Mg) nach Anspruch 1, wobei die Gusslegierung Folgendes enthält:
1,2 Gewichtsprozent Zink (Zn);
0,5 Gewichtsprozent Calcium (Ca);
0,5 Gewichtsprozent Mangan (Mn); und
wobei der Rest Magnesium (Mg) ist.

3. Knochenfixierungsvorrichtungen auf Basis von Magnesium (Mg) nach Anspruch 1, wobei die Korrosionsrate der wärmebehandelten Legierung mindestens 40 % niedriger ist als die der unbehandelten Gusslegierung.

4. Knochenfixierungsvorrichtungen auf Basis von Magnesium (Mg) nach Anspruch 1, wobei die Korrosionsrate der wärmebehandelten Legierung mindestens 50 % niedriger ist als die der unbehandelten Gusslegierung.

5. Verfahren zur Herstellung patientenspezifischer bioresorbierbarer Knochenfixierungsvorrichtungen auf Basis von Magnesium (Mg), umfassend die folgenden Schritte:
Herstellen von patientenspezifischen Fixierungsvorrichtungen aus einer Legierung aus:
0,75 bis 2,0 Gewichtsprozent Zink (Zn);
0,25 bis 1,0 Gewichtsprozent Kalzium (Ca);
0,25 bis 0,75 Gewichtsprozent Mangan (Mn);
wobei der Rest Magnesium (Mg) ist; und
wobei das Zn/Ca-Atomverhältnis der hergestellten Legierung im Bereich von 1,2 bis 1,47 liegt,
Wärmebehandeln der Legierung,
wobei die Legierung durch Gießen hergestellt wird, und
wobei die Gusslegierung zu Pulver zermahlen wird und patientenspezifische Fixierungsvorrichtungen durch 3D-Druck hergestellt werden.

6. Verfahren nach Anspruch 5, wobei der Wärmebehandlungsschritt eine Lösungsbehandlung, Abschreckung und Alterungshärtung umfasst.

7. Verfahren nach Anspruch 5, wobei die wärmebehandelte Legierung verbesserte mechanische Eigenschaften und Biokorrosionseigenschaften aufweist.

8. Verfahren nach Anspruch 6, wobei die Alterungshärtung 2 bis 5 Stunden lang bei 200 °C durchgeführt wird und die wärmebehandelte Legierung die halbe Korrosionsrate der Gusslegierung aufweist.

9. Verfahren nach Anspruch 6, wobei die Alterungshärtung 3 bis 5 Stunden lang bei 200 °C durchgeführt wird und die wärmebehandelte Legierung die halbe Korrosionsrate der Gusslegierung aufweist.

10. Verfahren nach Anspruch 7, wobei die wärmebehandelte Legierung mit optimalen mechanischen Eigenschaften und Biokorrosionseigenschaften zusätzlich mit einer Keramikbeschichtung unter Verwendung eines Mikrolichtbogenoxidationsverfahrens beschichtet wird.

11. Verfahren nach Anspruch 9, wobei die Legierung biokompatible Korrosionsnebenprodukte aus Hydroxylapatit (HA) und Magnesiumhydroxid (Mg(OH)₂) aufweist.

12. Knochenfixierungsvorrichtungen auf Basis von Magnesium (Mg), die mit dem Verfahren nach Anspruch 8 oder 9 erhalten werden können.

## Revendications

1. Matériel de fixation osseuse à base de magnésium (Mg) biorésorbable spécifique d'un patient fait d'un alliage moulé constitué de :
0,75 à 2,0 pour cent en poids de zinc (Zn) ;
0,25 à 1,0 pour cent en poids de calcium (Ca) ;
0,25 à 1,0 pour cent en poids de manganèse (Mn) ;
le reste étant du magnésium (Mg),
le rapport atomique Zn/Ca de l'alliage préparé se situant dans la plage de 1,2 à 1,47 ; et l'alliage moulé étant traité à la chaleur et atomisé en poudre et le matériel de fixation spécifique d'un patient étant produit par impression 3D.

2. Matériel de fixation osseuse à base de magnésium (Mg) selon la revendication 1, dans lequel l'alliage moulé contient :
1,2 pour cent en poids de zinc (Zn) ;
0,5 pour cent en poids de calcium (Ca) ;
0,5 pour cent en poids de manganèse (Mn) ; et
le reste étant du magnésium (Mg).

3. Matériel de fixation osseuse à base de magnésium (Mg) selon la revendication 1, dans lequel le taux de corrosion de l'alliage traité à la chaleur est au moins 40 % inférieur à celui de l'alliage comme moulé non traité.

4. Matériel de fixation osseuse à base de magnésium (Mg) selon la revendication 1, dans lequel le taux de corrosion de l'alliage traité à la chaleur est au moins 50 % inférieur à celui de l'alliage comme moulé non traité.

5. Procédé de production de matériel de fixation osseuse à base de magnésium (Mg) biorésorbable spécifique d'un patient comprenant les étapes consistant à :
produire le matériel de fixation spécifique d'un patient constitué d'un alliage de :
0,75 à 2,0 pour cent en poids de zinc (Zn) ;
0,25 à 1,0 pour cent en poids de calcium (Ca) ;
0,25 à 0,75 pour cent en poids de manganèse (Mn) ;
le reste étant du magnésium (Mg) ; et
le rapport atomique Zn/Ca de l'alliage préparé se situant dans la plage de 1,2 à 1,47 ;
traiter à la chaleur l'alliage,
l'alliage étant produit par moulage, et
l'alliage moulé étant atomisé en poudre et le matériel de fixation spécifique d'un patient étant produit par impression 3D.

6. Procédé selon la revendication 5, dans lequel l'étape de traitement à la chaleur est un traitement de solution, une extinction ou un durcissement à l'âge.

7. Procédé selon la revendication 5, dans lequel l'alliage traité à la chaleur possède des propriétés mécaniques et de biocorrosion améliorées.

8. Procédé selon la revendication 6, dans lequel le durcissement à l'âge est réalisé durant 2 à 5 heures à 200 °C et l'alliage traité à la chaleur possède la moitié du taux de corrosion de l'alliage comme moulé.

9. Procédé selon la revendication 6, dans lequel le durcissement à l'âge est réalisé durant 3 à 5 heures à 200 °C et l'alliage traité à la chaleur possède la moitié du taux de corrosion de l'alliage comme moulé.

10. Procédé selon la revendication 7, dans lequel l'alliage traité à la chaleur avec des propriétés mécaniques et de biocorrosion optimales est en outre revêtu avec un revêtement en céramique en utilisant un procédé d'oxydation à micro arc.

11. Procédé selon la revendication 9, dans lequel l'alliage a des sous-produits de corrosion biocompatibles d'hydroxyapatite (HA) et d'hydroxyde de magnésium (Mg(OH)₂).

12. Matériel de fixation osseuse à base de magnésium (Mg) que l'on peut obtenir avec le procédé de la revendication 8 ou 9.
